# EUROPEAN PATENT SPECIFICATION

(11) **EP 3 787 736 B1**
(45) Date of publication and mention of the grant of the patent: **03.08.2022**
(21) Application number: 19724260.5
(22) Date of filing: 01.05.2019
(51) Int. Cl.: A61N 1/36, A61F 2/16

(54) **RETINAL IMPLANT FIXATION APPARATUS AND TECHNIQUES**
VORRICHTUNG UND VERFAHREN ZUR FIXATION VON NETZHAUTIMPLANTATEN
APPAREIL ET TECHNIQUES DE FIXATION D'IMPLANT RÉTINIEN

(30) Priority: 02.05.2018 US 201862665652 P
(43) Date of publication of application: 10.03.2021
(73) Proprietor: Nano-Retina Ltd., 4673308 Herzliya (IL)
(72) Inventor: MENDELEWICZ, Rani, 4632111 Herzliya (IL); RIGLER, David, 6092000 Kadima-Tzoran (IL); GROSS, Yossi, 73160 Moshav Mazor (IL); SAAR, Ben, 4341721 Ra'anana (IL); MILSTAIN, Yaakov, 3094001 Zichron Yaakov (IL); RAZ PRAG, Dorit, 3707767 Pardes Hanna-Karkur (IL); DEGTIAR, Boris, 7173798 Modi'in (IL); SHAKI, Daniel, 6424406 Tel Aviv (IL); RIMER-COHEN, Ofir, Tslafon, 9975000 (IL); CZACZKES, Tsachi, 3706227 Pardes Hanna-Karkur (IL)
(74) Representative: Evens, Paul Jonathan
(86) International application number: PCT/IL2019/050484
(87) International publication number: WO 2019/211847

(56) References cited:
- WO-A2-2014/141089
- US-A1- 2010 121 444
- US-A1- 2011 313 522

## Description

### FIELD OF THE INVENTION

The present invention relates generally to implantable medical devices, and specifically to a retinal prosthesis.

### BACKGROUND OF THE INVENTION

Retinal deficiencies, due to degenerative retinal diseases, are a leading cause of blindness and visual impairment. Implantation of a retinal prosthesis is a technology for restoring some useful vision in individuals suffering from retina-related blindness.

The retina is a multi-layered light-sensitive structure that lines the inner part of the eye. The retina contains photoreceptor cells, for example rods and cones, which capture light and convert light signals into neural signals in the other retinal layers transmitted through the optic nerve to the brain. Rods are responsible for light sensitive, low resolution and night vision, whereas cones are responsible for sharp, high resolution color vision. The cones' highest concentration is in the fovea, which defines the center of the visual axis on the retina, and which allows for maximum acuity of vision.

WO 2014/141089 relates to a retinal prosthesis, and discloses apparatus according to the pre-characterizing portion of appended claim 1.

### SUMMARY OF THE APPLICATION

In accordance with the present invention, there is provided apparatus as defined in appended independent claim 1. Embodiments of the present invention are defined in appended claims dependent on independent claim 1. Methods of deploying the apparatus in an eye of a subject are described as a way to understand how the apparatus may be used, but do not form part of the present invention. Aspects, embodiments and examples disclosed herein which do not fall within the scope of the appended claims do not form part of the invention, and are merely provided for illustrative purposes.

In some applications of the present invention, apparatus is provided for securing a retinal implant in an eye of a subject. Typically, an anchor is coupled to the retinal implant and is configured to anchor the retinal implant to the retina such that when the implant is implanted on the retina, an anterior portion of the anchor is mounted in an anterior segment of the eye, e.g., in a posterior chamber, typically against a ciliary sulcus of the eye.

The retinal implant is configured to stimulate the retina of the subject suffering from a retinal disease in order to restore at least partial vision in the subject. For some applications, the retinal implant comprises an electrode array for stimulation of the retina, and, optionally, an array of photosensors and/or driving circuitry, configured to receive signals from the photosensors and in response thereto, to drive the electrodes to apply currents to the retina. The retinal implant is typically placed on the retina in an epi-retinal position, and the electrode array typically contacts the retina.

In accordance with some applications of the present invention, the anchor is shaped to define an anterior portion, a posterior portion coupled to and surrounding the retinal implant, and a middle portion extending longitudinally between the anterior portion and the posterior portion. Typically, the middle portion is shaped to define an elastic element, e.g., a helical spring. When the anchor is fully deployed in the eye, the anterior portion is anchored against an anterior segment of the eye, and the elastic element extends longitudinally from the anterior of the eye to the posterior of the eye such that the retinal implant, surrounded by the posterior portion of the anchor, is placed on the retina.

Typically, the apparatus in a fully-compressed state is inserted into the eye through a limbal incision. In the fully-compressed state, the middle portion of the anchor is longitudinally compressed such that a distance between the anterior portion and the posterior portion of the anchor is smaller than a distance between the anterior portion and the posterior portion of the anchor when the apparatus is in an expanded state thereof. For some applications, the apparatus is compressed by a first compressing element, e.g., a first compressing suture, which maintains the apparatus in a compressed state by securing the anterior portion with respect to the posterior portion.

Subsequently to inserting the apparatus into the eye, the anterior portion is anchored to the anterior segment of the eye, e.g., in the posterior chamber, typically against the ciliary sulcus of the eye, typically through at least one haptic, e.g., two haptics, which are coupled to the anterior portion of the anchor. For some applications, the haptics are sutured to the ciliary sulcus of the eye. Anchoring the anterior portion of the anchor to the ciliary sulcus typically stabilizes the retinal implant in the eye and allows subsequent full deployment of the anchor, as described below, and stable implantation of the retinal implant on the retina.

Subsequently to the anchoring of the anterior portion of the anchor, a first portion of the anchor is released by uncoupling the anterior portion of the anchor from the posterior portion of the anchor (typically by removing the first compressing element, e.g., the first compressing suture, that secures the anterior portion of the anchor to the posterior portion of the anchor). Upon removal of the first compressing element, e.g., the first compressing suture, the elastic element is partly released such that a portion of elastic element is extended longitudinally into a vitreous cavity of the eye. Typically, following removal of the first compressing element, e.g., the first compressing suture, a distance of the anterior portion to the posterior portion of the anchor is 30 - 50% of the distance of the anterior portion to the posterior portion of the anchor following deployment in the eye. Following removal of the first compressing element, e.g., the first compressing suture, the apparatus remains in a partly-compressed state in which a second compressing element, e.g., a second compressing suture, maintains the apparatus in a partly-compressed state by securing a section of the elastic element to the posterior portion.

Subsequently to releasing the first portion of the anchor, the posterior portion of the anchor is grasped by a grasping tool and a second portion of the anchor is released by uncoupling a section of the elastic element from the posterior portion of the anchor (typically by removing the second compressing element, e.g., the second compressing suture, that secures the section of the elastic element to the posterior portion). Removal of the second compressing element, e.g., the second compressing, suture allows extending of the anchor longitudinally to a full implantation length thereof, while lowering the retinal implant onto the retina. Typically, the retinal implant is implanted in an epi-retinal position, such that the electrodes contact the retina and provide stimulation to the retina. Typically, the surgeon has full manual control of the release of the retinal implant during the entire implantation procedure, from the compressed state to the deployed state.

The present invention will be more fully understood from the following detailed description of embodiments thereof, taken together with the drawings, in which:

### BRIEF DESCRIPTION OF THE DRAWINGS

Figs. 1A, 1B, and 1C are schematic illustrations of apparatus, in an unconstrained state thereof, for securing a retinal implant in an eye of a subject, in accordance with some applications of the present invention;
Fig. 2 is a schematic illustration of components of the apparatus for securing the retinal implant in the eye of the subject, in accordance with some applications of the present invention;
Fig. 3 is a schematic illustration of the apparatus for securing the retinal implant in the eye of the subject, configured for insertion into the eye, in a compressed state thereof, in accordance with some applications of the present invention;
Figs. 4A-G are schematic illustrations of a cross section of the eye showing the apparatus being deployed in the eye;
Fig. 5 is a schematic illustration of a view of the apparatus for securing a retinal implant in an eye of a subject, fully deployed in the eye;
Figs. 6A and 6B are schematic illustrations of rotational-force reduction of the apparatus;
Figs. 7A-C are schematic illustrations of another apparatus, in an unconstrained state thereof, for securing a retinal implant in an eye of a subject, in accordance with some applications of the present invention;
Fig. 8 is a schematic illustration of components of the apparatus of Figs. 7A-C, in accordance with some applications of the present invention;
Figs. 9A-B are schematic illustrations of the apparatus of Figs. 7A-C in a constrained state thereof, in accordance with some applications of the present invention;
Figs. 10A-F are schematic illustrations of a surgical procedure for implantation of the apparatus of Figs. 7A-C;
Fig. 11 is a schematic illustration of a front view of an eye having the apparatus of Figs. 7A-C deployed in the eye; and
Figs. 12A-B are schematic illustrations of an anchor, in an unconstrained state, in accordance with some applications of the present invention.

### DETAILED DESCRIPTION OF THE APPLICATION

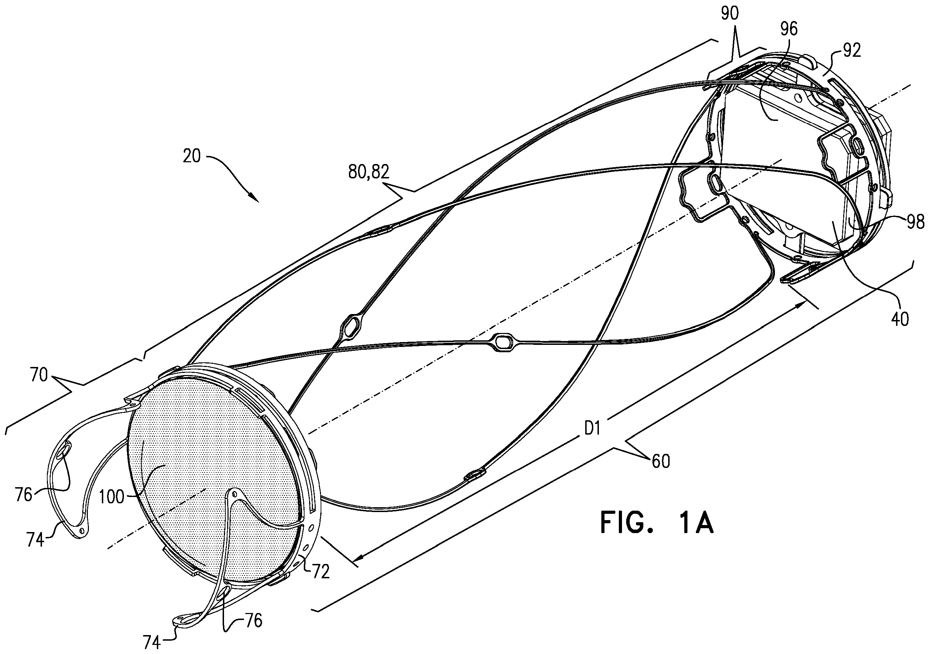

Reference is first made to Figs. 1A-C, which are schematic illustrations of apparatus 20 for securing a retinal implant 40 in an eye of a subject, in accordance with some applications of the present invention. Typically, apparatus 20 comprises an anchor 60 which is coupled to retinal implant 40 and is configured to anchor retinal implant 40 to a retina 306 in an epi-retinal position.

Anchor 60 is typically shaped to define (i) an anterior portion 70, (ii) a posterior portion 90 surrounding retinal implant 40, and (iii) a middle portion 80 extending longitudinally between anterior portion 70 and posterior portion 90. As shown in Figs. 1A-C, for some applications, anterior portion 70 comprises an anterior ring 72, posterior portion 90 comprises a posterior ring 92 surrounding retinal implant 40, and middle portion 80 comprises an elastic element 82 (e.g., a helical spring) extending longitudinally between anterior ring 72 and posterior ring 92.

Figs. 1A-C show apparatus 20 in an unconstrained state. When expanded and unconstrained, elastic element 82 typically has an unconstrained length that is at least 20 mm and/or less than 40 mm, e.g., between 20 - 40 mm, e.g., between 18 - 35 mm. Typically, the length of elastic element 82 is also a distance D1 between anterior ring 72 and posterior ring 92 (e.g., between 13 and 23 mm). In this context, in the specification and in the claims, a "distance" refers to the distance from a first closest surface to a second closest surface.

Optionally, the struts of elastic element 82 are coated with a material configured to prevent the buildup of fibrin or other biological material on the struts.

For some applications, anterior portion 70 further comprises at least one haptic 74, e.g., two haptics 74. Typically, anterior portion 70 is configured to be anchored against an anterior segment 308 of the eye by anchoring haptics 74 to the eye. For some applications, haptics 74 are anchored to the eye by expanding and applying a force to the eye. Additionally, or alternatively, haptics 74 are anchored to anterior segment 308 of the eye by passing a haptic-suture through holes 76, and subsequently suturing haptics 74 to the eye. Typically, a maximum distance between haptics 74 once implanted is 11 - 13.5 mm. Typically, anterior portion 70 (e.g., haptics 74) is flexible in order to allow or facilitate placement of the anterior portion (e.g., the haptics) within the eye. Typically, the thickness and/or shape of haptics 74 are configured to prevent excessive pressure on the eye tissue and/or accommodate a range of eye sizes; for example, haptics 74 may be elastic in order to provide these properties.

For some applications, an intraocular lens (IOL) 100 is coupled to anterior portion 70 of anchor 60. IOL 100 is typically fitted into anterior portion 70 following deployment of apparatus 20 in the eye of the subject.

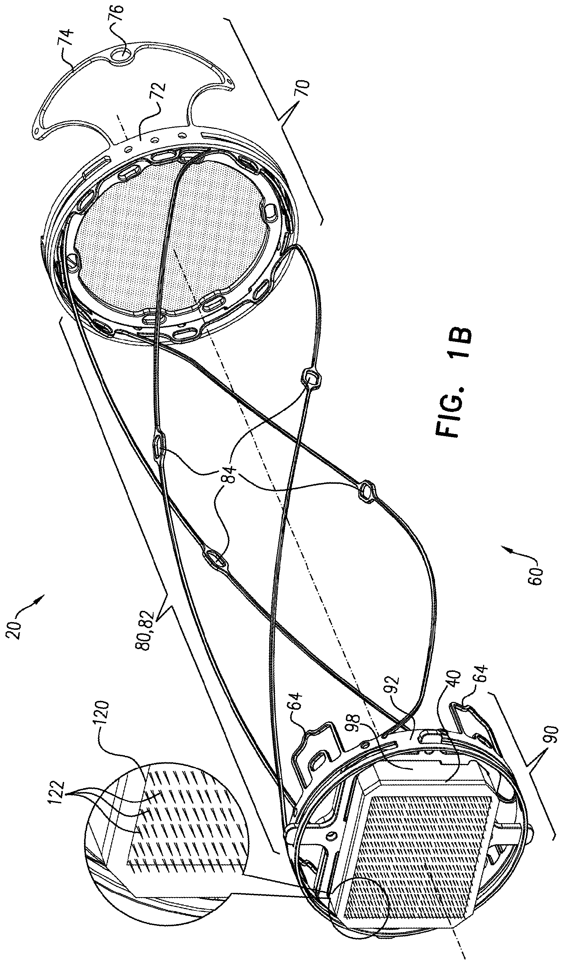

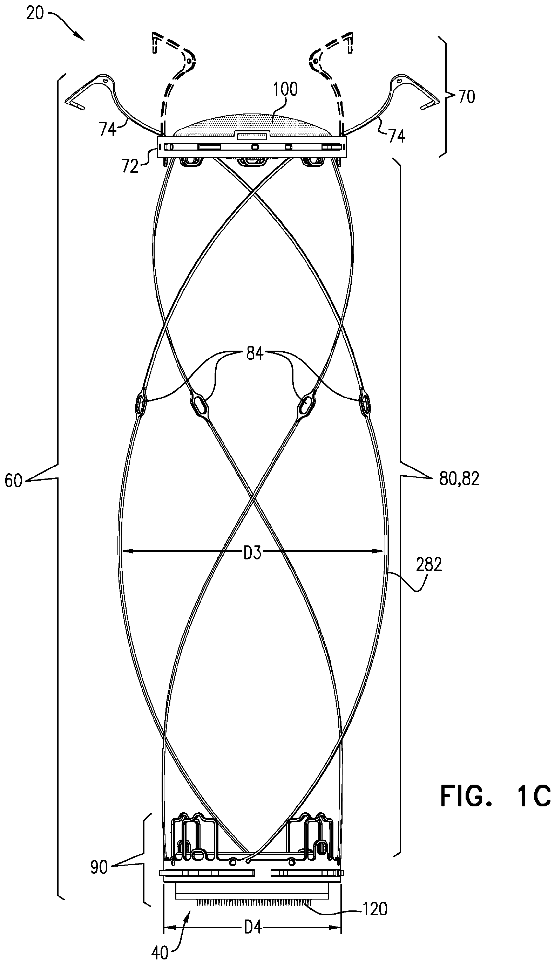

Typically, posterior portion 90 surrounds retinal implant 40. Retinal implant 40 comprises an electrode array 120 comprising electrodes 122 which are configured to stimulate retina 306 by applying currents to the retina. A view of electrode array 120 is shown in Figs. 1B and 1C. For some applications (as illustrated in Fig. 1B and 1C) electrodes 122 are penetrating electrodes configured to penetrate the retina. Alternatively, electrode array 120 comprises surface electrodes which contact the retina but do not penetrate the retina.

For some applications, such as shown in the figures, retinal implant 40 additionally comprises an array of photosensors 96 and driving circuitry 98 configured to receive signals from photosensors 96 and in response thereto, to drive electrodes 122 to apply currents to the retina. Alternatively, retinal implant 40 does not comprise the array of photosensors 96, and photosensors 96 are instead disposed outside of the eye, e.g., as an element of an extraocular imaging device (i.e., a camera), for example using techniques described in US Patent Application Publication 2014/0222103 to Lauritzen et al. and/or US Patent 6,458,157 to Suaning. Alternatively or additionally, retinal implant 40 does not comprise driving circuitry 98 or comprises only minimal circuitry. For example, techniques may be used that are described in US Patent 5,024,223 to Chow and/or US Patent 5,895,415 to Chow et al..

Anchor 60 typically anchors retinal implant 40 such that when anchor 60 is fully deployed in the eye, anterior portion 70 is anchored against anterior segment 308 of the eye, and elastic element 82 extends longitudinally from the anterior of the eye to the posterior of the eye such that retinal implant 40, surrounded by posterior portion 90 of anchor 60, is stably placed on the retina.

Reference is made to Fig. 1C, which is a schematic illustration of an additional configuration of apparatus 20, in accordance with some applications of the present invention. For some applications, elastic element 82 is pre-arched, so as to form a rounded bulge as shown in Fig. 1C. Thus, as shown, a distance D3 of helical legs 282 of elastic element 82 in the center of element 82 is larger than a distance D4 of helical legs 282 of elastic element 82 in the posterior portion of elastic element 82, forming a barrel-like shape. For example, D3 may be 5 - 20% larger than D4. Typically, the rounded bulge (barrel-like shape) structure of elastic element 82 enhances flexibility of anchor 60 and facilitates longitudinal compression of the anchor without buckling.

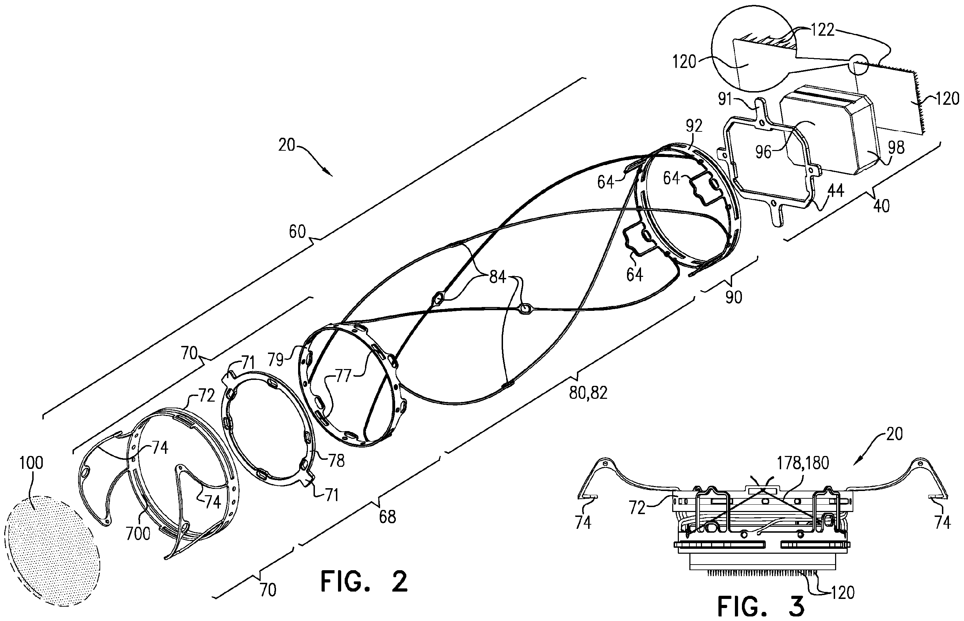

Reference is now made to Fig. 2, which is a schematic illustration of components of the apparatus for securing the retinal implant in the eye of the subject, in accordance with some applications of the present invention. As shown in Fig. 2 and described hereinabove with reference to Figs. 1A-C, apparatus 20 comprises anchor 60 and retinal implant 40, which is surrounded by anchor 60. As shown, anchor 60 comprises an anterior portion 70, posterior portion 90, and a middle portion 80 between anterior portion 70 and posterior portion 90. Anchor 60 is configured to anchor implant 40 on to retina 306 of the subject by stabilizing anterior portion 70 to anterior segment 308 of the eye and positioning posterior portion 90 on the retina.

As shown in Fig. 2, anterior portion 70 comprises an anterior ring 72 and two haptics 74, which are coupled to anterior ring 72 and configured to anchor anterior portion 70 to anterior segment 308 of the eye. Typically, as shown in Figs. 1C, 3, and 4B, haptics 74, when unconstrained, extend radially outward from anterior ring 72 in order to anchor anterior portion 70 within a posterior chamber 309 of the eye, typically against a ciliary sulcus 310 of the eye.

Typically, anterior ring 72 has an outer diameter of at least 4 mm, no more than 8 mm, and/or between 4 and 8 mm, such as at least 6 mm, no more than 7 mm, and/or between 6 and 7 mm, which is smaller than the diameter of the eye at the transverse section passing through ciliary sulcus 310.

For some applications, in addition to anterior ring 72, anterior portion 70 additionally comprises a rotational-force reduction element 68 at an anterior end of elastic element 82 comprising a first rotational-force reduction ring 79 and a second rotational-force reduction ring 78, coupled to first rotational-force reduction ring 79 and inhibited from rotation with respect to first rotational-force reduction ring 79.

Typically, rotational-force reduction element 68 is configured to rotate and to reduce a rotational force stored in elastic element 82 that might otherwise be applied to retinal implant 40 during implantation. In other words, rotational-force reduction element 68 typically allows some rotation of elastic element 82, while retinal implant 40 remains stationary. Typically, elastic element 82 extends from first rotational-force reduction ring 79 to posterior portion 90 of anchor 60.

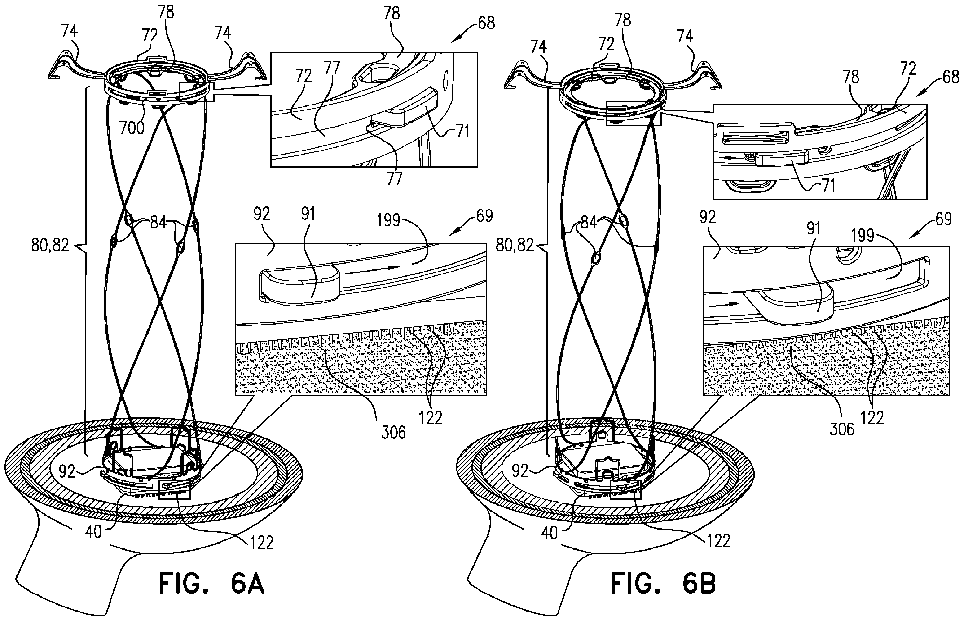

Typically, when anchor 60 is in the assembled state (as illustrated in Figs. 6A-B), second rotational-force reduction ring 78 is surrounded by first rotational-force reduction ring 79 and is inhibited from rotating with respect to first rotational-force reduction ring 79. Typically, one or more protrusions 71 from second rotational-force reduction ring 78 are inserted into holes 77 in first rotational-force reduction ring 79 such that second rotational-force reduction ring 78 is locked and cannot rotate with respect to first rotational-force reduction ring 79. Anterior ring 72 surrounds rotational-force reduction element 68, such that protrusions 71 are inserted into slits 700 of anterior ring 72, allowing rotation of rotational-force reduction element 68 with respect to anterior ring 72. Rotation of rotational-force reduction element 68 with respect to anterior ring 72 allows the relieving of rotational forces in elastic element 82 that might otherwise cause rotation of implant 40 during and following implantation on the retina.

Reference is now made to Fig. 3, which is a schematic illustration of apparatus 20 in a fully-compressed state thereof. Typically, apparatus 20 is inserted into the eye of the subject in the fully-compressed state. In the fully-compressed state of apparatus 20, elastic element 82 is longitudinally compressed such that a distance between anterior portion 70 and posterior portion 90 is smaller than a distance between anterior portion 70 and posterior portion 90 in an expanded state of apparatus 20 (for example as shown in Figs. 1A-C).

Apparatus 20 is maintained in the fully-compressed state by at least one compressing element, e.g., a suture and/or a clip. The at least one compressing element includes a first compressing element 178, such as a first compressing suture 180, which is used to compress apparatus 20. For some applications, first compressing element 178, e.g., first compressing suture 180, is configured to secure anterior portion 70 with respect to posterior portion 90 to compress apparatus 20, such that a distance of anterior portion 70 to posterior portion 90 is 2.5 - 4 mm.

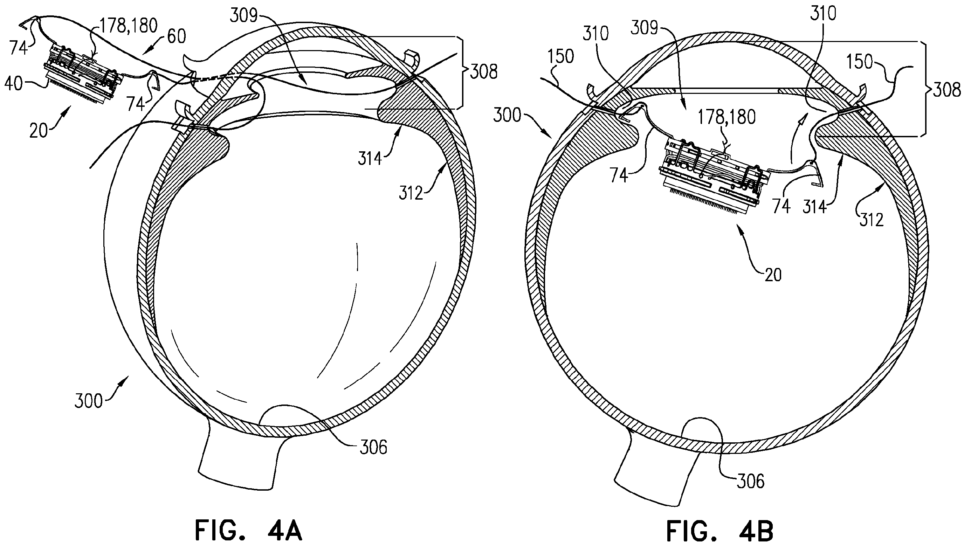

Figs. 4A-G are schematic illustrations of a surgical procedure for implantation of apparatus 20. Figs. 4A-G show a cross section of eye 300 and the deployment of apparatus 20 in eye 300 to yield the implantation and anchoring of retinal implant 40 on retina 306.

Reference is first made to Fig. 4A. As shown, apparatus 20 is inserted into eye 300 in a fully-compressed state of apparatus 20. During insertion of apparatus 20 into the eye, apparatus 20 is maintained in the fully-compressed state by first compressing element 178, e.g., first compressing suture 180, which secures anterior portion 70 with respect to posterior portion 90. Apparatus 20 is typically inserted through a limbal incision in the eye, typically following removal of the native lens and vitreous body of the eye. For some applications, apparatus 20 is inserted through the limbal incision by grasping the apparatus by one of haptics 74 and pushing the apparatus through the limbal incision. Alternatively, an insertion tool is used to insert apparatus 20 through the limbal incision. For example, the insertion tool may comprise a cannula in which the apparatus is removably disposed. The cannula is inserted through the limbal incision and the apparatus is deployed from the cannula within the eye.

Reference is now made to Figs. 4B-C. Following insertion into the eye, apparatus 20 is anchored to anterior segment 308 of eye 300, typically within posterior chamber 309, typically against ciliary sulcus 310 of the eye. As mentioned above, for some applications, anchor 60 comprises two haptics 74, and each haptic 74 is placed against ciliary sulcus 310. For some applications, each haptic 74 is sutured to ciliary sulcus 310 by a haptic-suture 150. Anchoring haptics 74 to ciliary sulcus 310 typically stabilizes apparatus 20 in the eye and allows subsequent full deployment of anchor 60 and stable implantation of retinal implant 40 on retina 306. Alternatively, even though anchor 60 is configured to anchor retinal implant 40 to the retina such that anterior portion 70 of anchor 60 is mounted against ciliary sulcus 310, apparatus 20 may instead, typically inadvertently, be anchored to a pars plana of the eye, depending, for example, on the skill of the surgeon. (As is known, the pars plana includes the pars plana ciliaris 312 and the pars plicata ciliaris 314.)

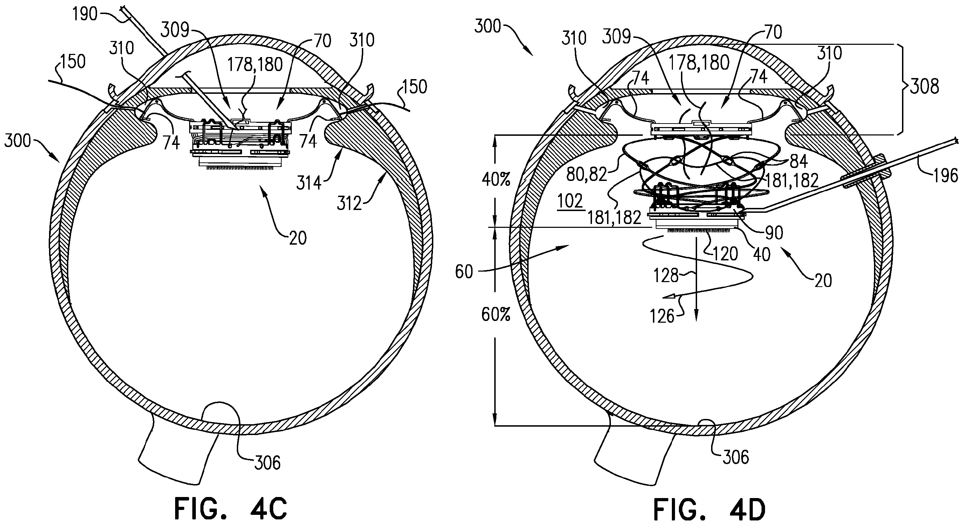

Reference is now made to Fig. 4D, which is a schematic illustration of partial release of the compression of apparatus 20. Typically, subsequently to the anchoring of anterior portion 70 to ciliary sulcus 310 through haptics 74 (as shown in Fig. 4C), a first portion of anchor 60 is released by separating anterior portion 70 of anchor 60 from posterior portion 90 of anchor 60. This separation is typically facilitated by removal of first compressing element 178, e.g., first compressing suture 180, e.g., by cutting first compressing suture 180 using a cutting tool 190. Upon the cutting of first compressing element 178, e.g., first compressing suture 180, elastic element 82 is partly released, such that a portion of elastic element 82 is extended longitudinally, posteriorly, into a vitreous cavity 102 of eye 300. As shown in Fig. 4D, following the cutting of first compressing element 178, e.g., first compressing suture 180, an anterior portion of elastic element 82 is decompressed and a posterior portion of elastic element 82 remains compressed.

Typically, following the cutting of first compressing element 178, e.g., first compressing suture 180, a distance of anterior portion 70 of anchor 60 to posterior portion 90 of anchor 60, is 30 - 50%, e.g., 40%, of the distance of anterior portion 70 to posterior portion 90 of anchor 60 following deployment of apparatus 20 in the eye.

Following removal of first compressing element 178, e.g., first compressing suture 180, the apparatus remains in a partly-compressed state, shown in Fig. 4D, in which a second compressing element 181, e.g., a second compressing suture 182, partly compresses apparatus 20 by coupling a section of elastic element 82 to posterior portion 90 of the anchor 60. Fig. 4D shows second compressing suture 182 in a slightly loose state for illustration purposes (typically, the section of elastic element 82 and posterior portion 90 are more tightly compressed in the partly-compressed state). In the partly-compressed state, a distance between anterior portion 70 and posterior portion 90 (a) is larger than a distance between anterior portion 70 and posterior portion 90 in the fully-compressed state of apparatus 20, and (b) is smaller than a distance between anterior portion 70 and posterior portion 90 in the expanded state, i.e., following deployment in the eye, of apparatus 20. Typically, in the partly-compressed state the distance of anterior portion 70 to posterior portion 90 is 8 - 15 mm.

Arrows 126 and 128 schematically indicate helical and posterior motion, respectively, of apparatus 20 following removal of first compressing element 178, e.g., first compressing suture 180, as apparatus 20 assumes the configuration shown in Fig. 4D.

It is noted that second compressing suture 182 is not shown in in Figs. 3-4C for clarity of illustration.

In the partly-compressed state, second compressing element 181, e.g., second compressing suture 182, typically passes through holes 84 in respective helical legs 282 of elastic element 82 and secures a section of elastic element 82 to posterior portion 90 of anchor 60. Thus, second compressing element 181, e.g., second compressing suture 182, maintains apparatus 20 in the partly-compressed state by securing a section of elastic element 82 to posterior portion 90 of anchor 60 as shown in Fig. 4D.

As shown in Fig. 4D, subsequently to partial release of anchor 60 as shown in the transition from Fig. 4C to Fig. 4D, posterior portion 90 of anchor 60, e.g., one or more loops 64, is grasped by a grasping tool 196 in order to allow subsequent full release of the compression of apparatus 20 and lowering of retinal implant 40 on to retina 306 in a controlled manner.

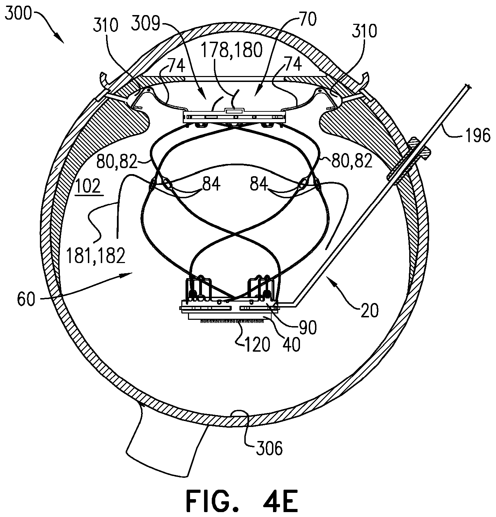

Reference is now made to Fig. 4E, which is a schematic illustration of apparatus 20 following the partial release of the compression of apparatus 20 and the deployment of apparatus 20 in eye 300. Subsequently to grasping posterior portion 90, while posterior portion 90 of anchor 60 is still grasped by grasping tool 196, the remainder of anchor 60 is released by facilitating the separating (from posterior portion 90) of the portion of elastic element 82 that was secured to posterior portion 90 of anchor 60 (as shown in Fig. 4D). Separating of the portion of elastic element 82 from posterior portion 90 is typically carried out by removing, e.g., by cutting, second compressing element 181, e.g., second compressing suture 182, that couples the portion of elastic element 82 to posterior portion 90. Cutting of second compressing element 181, e.g., second compressing suture 182, allows the extending of anchor 60 longitudinally, posteriorly, to a full deployed length thereof (constrained anteriorly by the ciliary sulcus and posteriorly by the retina). Fig. 4E shows second compressing element 181, e.g., second compressing suture 182, after it has been cut, while posterior portion 90 of anchor 60 is still grasped by grasping tool 196, preventing the full extending of anchor 60 longitudinal to the full deployed length thereof.

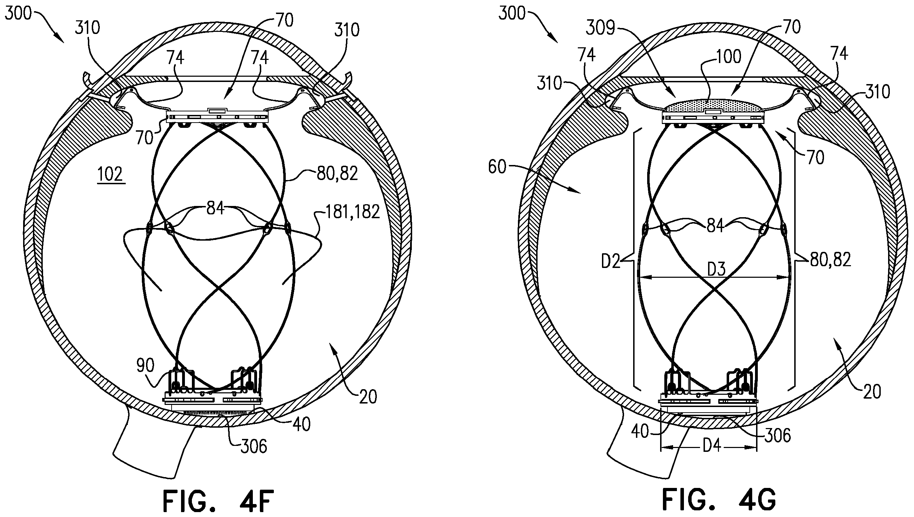

Reference is now made to Fig. 4F, which is a schematic illustration of apparatus 20 following the full release of the compression of apparatus 20 and the deployment of apparatus 20 in eye 300. Following removal of second compressing element 181, e.g., second compressing suture 182, retinal implant 40 is lowered onto retina 306 by the surgeon, using grasping tool 196. Optionally, the surgeon may use grasping tool 196 to maneuver retinal implant 40 slightly sideways during lowering, such as in order to compensate for the slight tilt of the visual axis relative to the optical axis of the eye. Typically, retinal implant 40 is implanted in an epi-retinal position such that electrode array 120 contacts retina 306 and provides stimulation to retina 306. (For some applications, upon full deployment of apparatus 20, respective central axes of anterior ring 72 and posterior ring 92 are angled with respect to each other, e.g., by about 5 degrees, because the retinal implantation site on the macula is positioned on the visual axis which is tilted by about 5 degrees with respect to the optical axis of the eye.)

As shown in Fig. 4F, when anchor 60 is fully deployed in eye 300, anterior portion 70 is anchored within posterior chamber 309, typically against ciliary sulcus 310, and elastic element 82 extends longitudinally from the anterior of eye 300 to the posterior of the eye, such that retinal implant 40, surrounded by posterior portion 90 of anchor 60, is placed on retina 306. For some applications, retinal implant 40 is placed on retina 306 such that electrodes 122 penetrate the retina. Alternatively, electrode array 120 comprises surface electrodes, which contact the retina but do not penetrate the retina. Posterior portion 90 of anchor 60 typically does not penetrate the retina.

Reference is now made to Fig. 4G, which is a schematic illustration of a cross section of eye 300, showing apparatus 20 in a fully deployed state in the eye, following insertion of intraocular lens (IOL) 100, in accordance with some applications of the present invention. For some applications, an intraocular lens (IOL) 100 is inserted into the eye and positioned in anterior ring 72, following deployment of anchor 60 in the eye.

In addition, when anchor 60 is deployed as shown in Fig. 4G, anchor 60 is in compression between the anterior and posterior of eye 300, and a distance D2 between anterior ring 72 and posterior ring 92 (i.e., a length of elastic element 82), is shorter than distance D1 when apparatus 20 is unconstrained (Fig. 1A). Typically, when deployed in the eye, anchor 60 presses in a posterior direction against retinal implant 40 and in an anterior direction against ciliary sulcus 310, (e.g., via contact of haptics 74 with the ciliary sulcus).

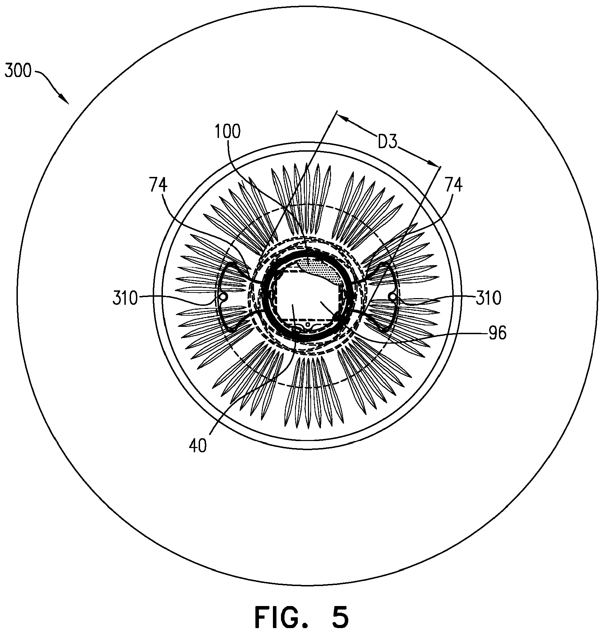

Reference is now made to Fig. 5, which is a schematic illustration of a front view of eye 300 having the apparatus for securing a retinal implant fully deployed in the eye. As shown, anchor 60, when deployed in the eye, allows generally unobstructed passage of light into the eye and to retinal implant 40 (in particular, unobstructed passage of light to photosensors 96 of retinal implant 40).

Reference is now made to Figs. 6A-B, which are schematic illustrations of rotational-force reduction of apparatus 20. Typically, but not necessarily, elastic element 82 of anchor 60 is rotated when compressed into the fully-compressed state of the apparatus, when anterior portion 70 and posterior portion 90 are brought closer together. Subsequently, during deployment of anchor 60 in the eye, apparatus described herein may be used to cause anchor 60 to rotate in the opposite direction, in order to relieve torsional forces stored in elastic element 82, while minimizing rotation of implant 40.

In order to reduce such torsional forces from causing rotation to implant 40 during and subsequently to implantation on the retina, as described hereinabove with reference to Fig. 2, anchor 60 additionally comprises a rotational-force reduction element 68 at an anterior end of elastic element 82. As described hereinabove, rotational-force reduction element 68 typically allows rotation of elastic element 82, while retinal implant 40 remains stationary, like a swivel mechanism. As described hereinabove with reference to Fig. 2, and shown in Figs. 6A-B, anterior ring 72 surrounds rotational-force reduction element 68, such that protrusions 71 are inserted into slits 700 of anterior ring 72, allowing rotation of rotational-force reduction element 68 with respect to anterior ring 72. For some applications, prior to removal of first compressing element 178, e.g., first compressing suture 180, as described hereinabove with reference to Fig. 4F, a rotation-controlling suture 184 is positioned to lock rotational-force reduction element 68 with respect to anterior ring 72, in order to inhibit rotation of rotational-force reduction element 68 with respect to anterior ring 72. Typically, following removal of first compressing element 178, e.g., first compressing suture 180, rotation-controlling suture 184 is removed and rotational-force reduction element 68 is thereby allowed to rotate with respect to anterior ring 72, to relieve torsional forces stored in elastic element 82. Alternatively, for some applications, first compressing element 178, e.g., first compressing suture 180, is additionally configured to lock rotational-force reduction element 68 with respect to anterior ring 72 in order to inhibit rotation of rotational-force reduction element 68 with respect to anterior ring 72. For such cases, upon removal of first compressing element 178, e.g., first compressing suture 180, rotational-force reduction element 68 is allowed to rotate with respect to anterior ring 72, to relieve torsional forces stored in elastic element 82.

For some applications, apparatus 20 additionally comprises posterior rotational-force reduction element 69 in posterior portion 90. Rotational-force reduction element 69 is typically configured to allow rotation of elastic element 82 while implant 40 remains stationary. Rotational-force reduction element 69 is generally the same as corresponding anterior rotational-force reduction element 68. Rotational-force reduction element 69 typically comprises a frame 44 (shown in Fig. 2), which surrounds retinal implant 40 and is shaped to define one or more protrusions 91, which pass through slit 199 in posterior ring 92 and is configured to rotate, thereby allowing rotation of elastic element 82, while implant 40 remains stationary. For some applications, rotation of rotational-force reduction element 69 occurs prior to placement of retinal implant 40 on the retina. Alternatively, rotation of rotational-force reduction element 69 occurs following placement of retinal implant on the retina.

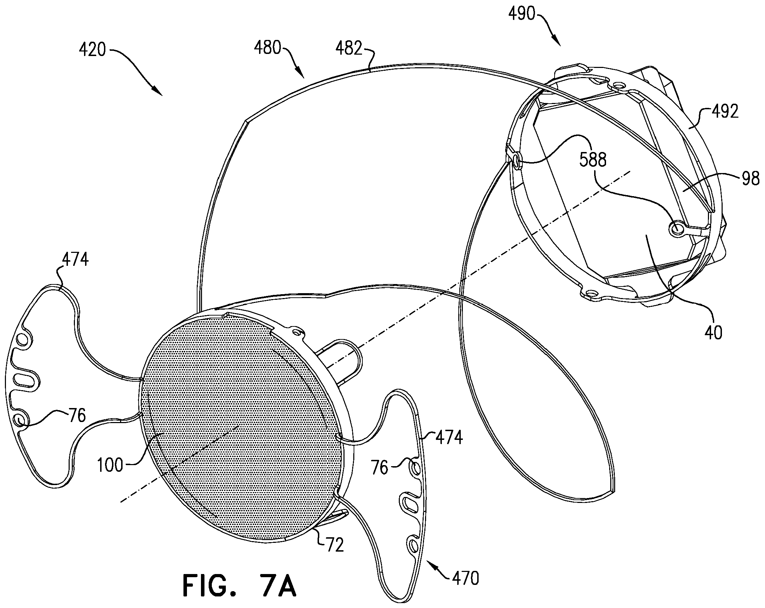

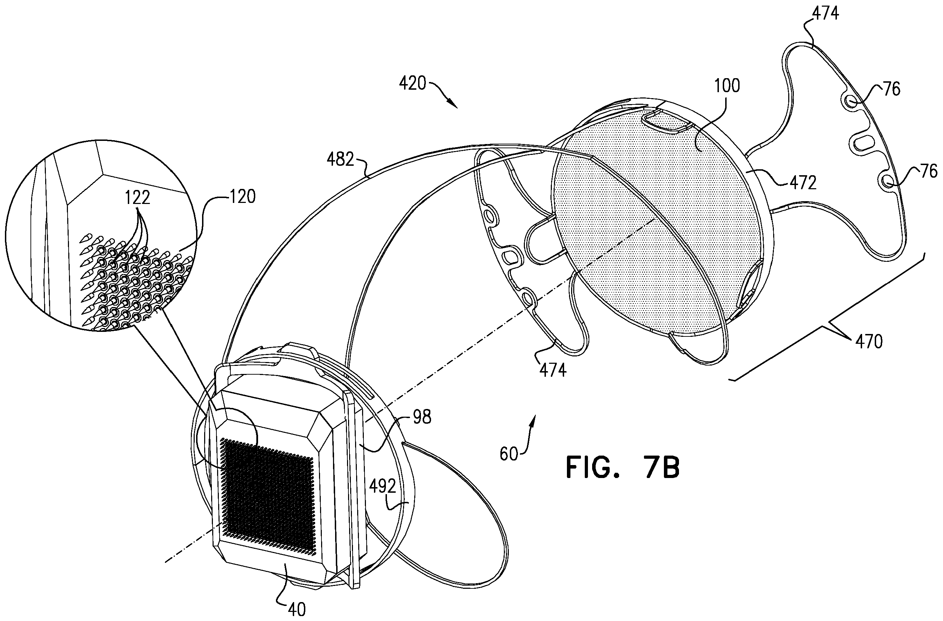

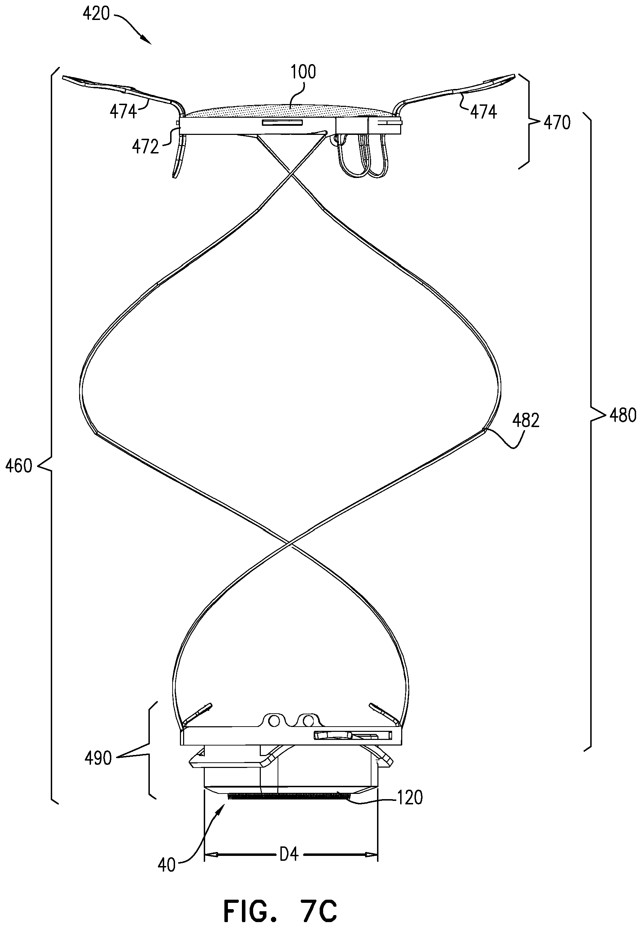

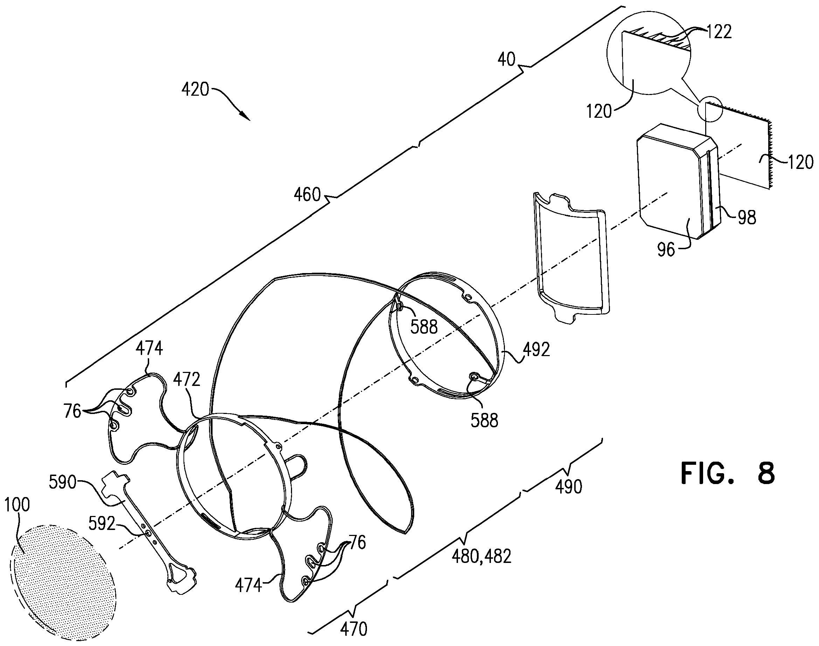

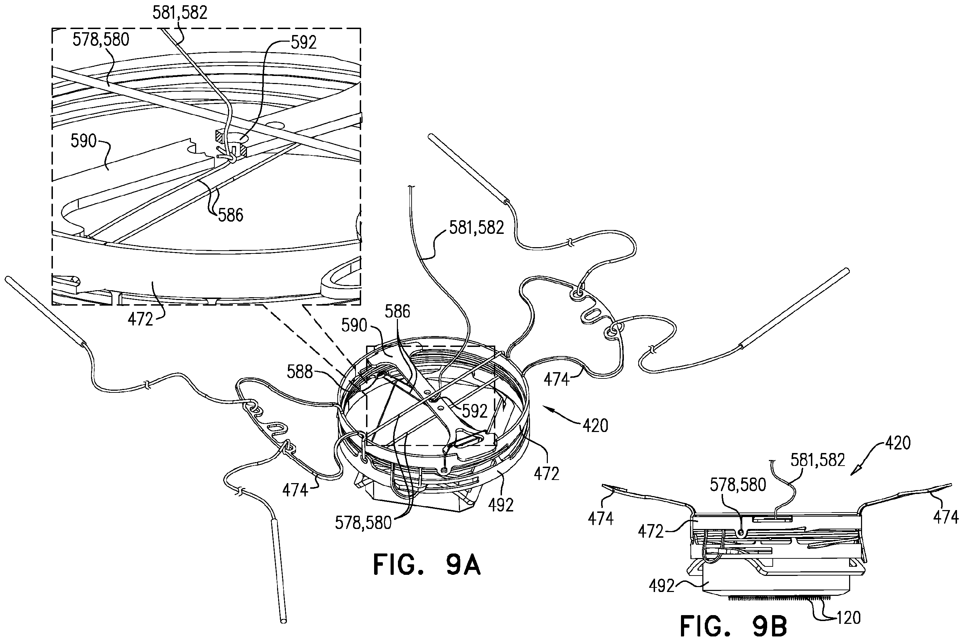

Reference is now made to Figs. 7A-C, which are schematic illustrations of apparatus 420, in an unconstrained state thereof, for securing retinal implant 40 in an eye of a subject, in accordance with some applications of the present invention. Reference is further made to Fig. 8, which is a schematic illustration of components of apparatus 420, in accordance with some applications of the present invention. Reference is still further made to Figs. 9A-B, which are schematic illustrations of apparatus 420 in a constrained state thereof, in accordance with some applications of the present invention. Other than as described below, apparatus 420 is similar to apparatus 20, described hereinabove with reference to Figs. 1A-6, and may implement any features thereof, *mutatis mutandis.* Like reference numerals refer to like elements. Apparatus 420 comprises an anchor 460 which is coupled to retinal implant 40 and is configured to anchor retinal implant 40 to retina 306 in an epi-retinal position, by stabilizing an anterior portion 470 of anchor 460 to anterior segment 308 of the eye and positioning a posterior portion 490 of anchor 460 on retina 306.

Anchor 460 is typically shaped to define (i) anterior portion 470, (ii) posterior portion 490 surrounding retinal implant 40, and (iii) a middle portion 480 extending longitudinally between anterior portion 470 and posterior portion 490. As shown in Figs. 7A-9B, for some applications, anterior portion 470 comprises an anterior ring 472, posterior portion 490 comprises a posterior ring 492 surrounding retinal implant 40, and middle portion 480 comprises an elastic element 482 (e.g., a spring) extending longitudinally between anterior ring 472 and posterior ring 492.

For some applications, anterior portion 470 further comprises at least one haptic 474, e.g., two haptics 474, which may be anchored to anterior segment 308 of the eye as described hereinabove regarding haptics 74. Typically, a maximum distance between haptics 474 once implanted is 11 - 13.5 mm. Typically, anterior ring 472 has an outer diameter of at least 4 mm, no more than 8 mm, and/or between 4 and 8 mm, such as at least 6 mm, no more than 7 mm, and/or between 6 and 7 mm, which is smaller than the diameter of the eye at the transverse section passing through ciliary sulcus 310.

For some applications, IOL 100 is coupled to anterior portion 470 of anchor 460. IOL 100 is typically fitted into anterior portion 470 following deployment of apparatus 420 in the eye of the subject.

Typically, posterior portion 490 surrounds retinal implant 40, which is described hereinabove with reference to Figs. 1B-C. Anchor 460 typically anchors retinal implant 40 such that when anchor 460 is fully deployed in the eye, anterior portion 470 is anchored against anterior segment 308 of the eye, and elastic element 482 extends longitudinally from the anterior of the eye to the posterior of the eye such that retinal implant 40, surrounded by posterior portion 490 of anchor 460, is stably placed on the retina.

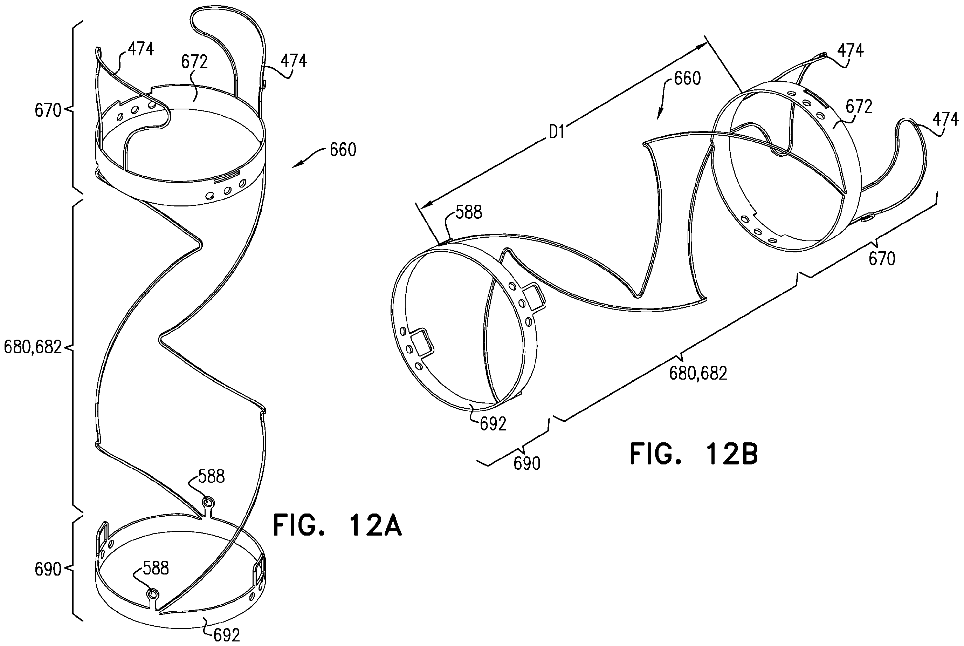

Reference is again made to Figs. 9A-B, which are schematic illustrations of apparatus 420 in a fully-compressed state thereof. Typically, apparatus 420 is inserted into the eye of the subject in the fully-compressed state. In the fully-compressed state of apparatus 420, elastic element 482 is longitudinally compressed such that a distance between anterior portion 470 and posterior portion 490 is smaller than a distance between anterior portion 470 and posterior portion 490 in an expanded state of apparatus 420 (for example as shown in Figs. 12A-B).

Apparatus 420 is maintained in the fully-compressed state by at least one compressing element, e.g., a suture and/or a clip. The at least one compressing element includes a first compressing element 578, such as a first compressing suture 580, which is used to compress apparatus 420. For some applications, first compressing element 578, e.g., first compressing suture 580, is configured to secure anterior portion 470 with respect to posterior portion 490 to compress apparatus 420, such that a distance of anterior portion 470 to posterior portion 490 is 2.5 - 4 mm.

In addition, second compressing element 581, e.g., a second compressing suture 582, is coupled to posterior portion 490, e.g., to posterior ring 492, such as during manufacture of apparatus 420. Optionally, second compressing suture 582 is coupled to posterior portion 490 via a posterior connecting suture 586 (e.g., second compressing suture 582 may be tied or otherwise coupled to posterior connecting suture 586). Posterior connecting suture 586 may be looped through hooks or rings 588 of posterior portion 490. Alternatively, second compressing suture 582 is directly coupled to posterior portion 490, such as by having a portion of second compressing suture 582 looped through hooks or rings 588.

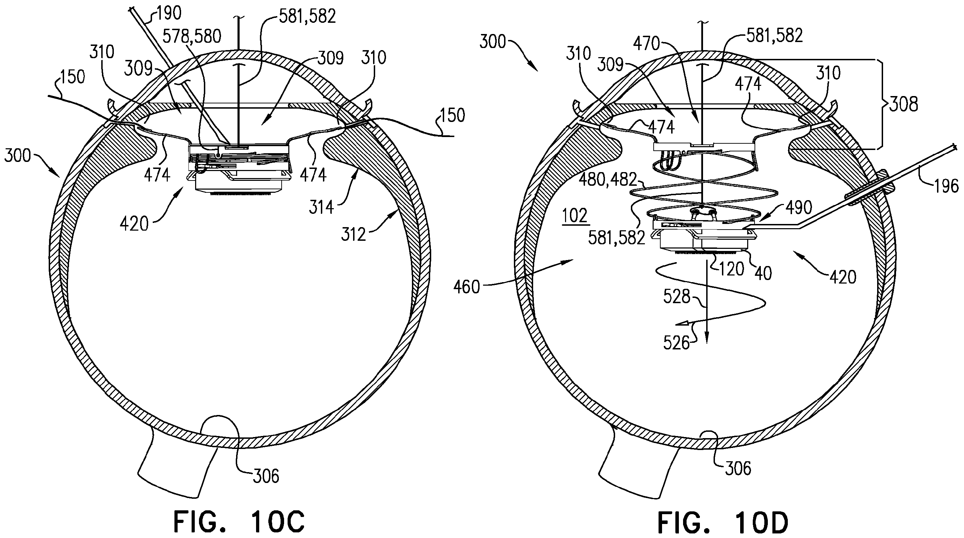

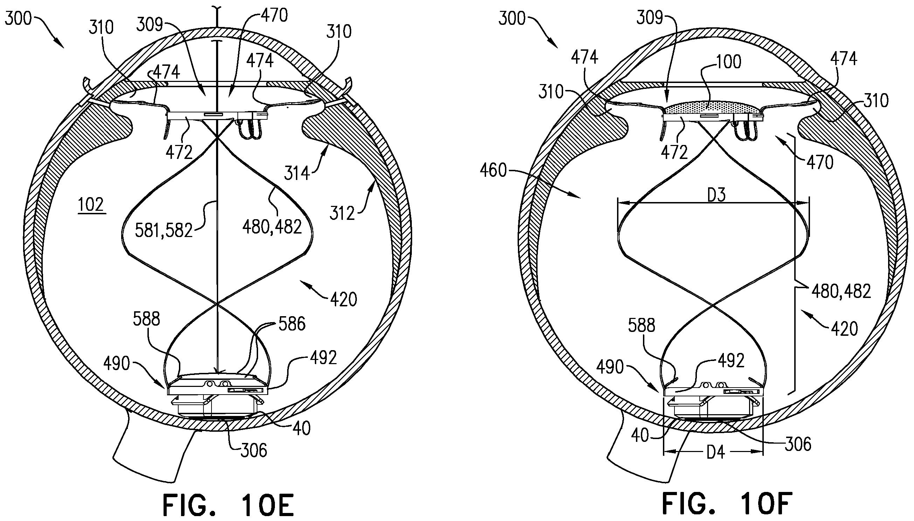

For some applications, a second-compressing-suture balancing element 590 is provided that is removably coupled (e.g., by one or more sutures) to anterior portion 470, e.g., anterior ring 472. (Second-compressing-suture balancing element 590 is shown in broken view in the enlargement in Fig. 9A to more clearly show the coupling thereto of second compressing suture 582.) For example, second-compressing-suture balancing element 590 may be shaped as a crossbar (as shown) or another shape, e.g., a ring (configuration not shown). Typically, second-compressing-suture balancing element 590 is shaped so as to define a central opening 592 through which second compressing suture 582 passes. As a result, second-compressing-suture balancing element 590 helps maintain second compressing suture 582 centered and balanced when second compressing suture 582 is held taut during the implantation procedure, as described hereinbelow with reference to Fig. 10D, and as posterior portion posterior portion 490 is lowered onto the retina, as described hereinbelow with reference to Fig. 10E. Before completion of the implantation procedure, second-compressing-suture balancing element 590 is removed, such as by cutting the one or more sutures that couple it to anterior portion 470, typically soon before or after removing second compressing suture 582.

For some applications, respective portions of one or more of the sutures described hereinabove are coupled (e.g., tied) to second-compressing-suture balancing element 590. Removal of second-compressing-suture balancing element 590, as described hereinbelow with reference to Fig. 10E, removes from the eye all of the one or more sutures coupled thereto. For example, one or more of the following sutures may be coupled to second-compressing-suture balancing element 590: first compressing suture 580, second compressing suture 582, posterior connecting suture 586, and/or the one or more sutures that removably couple second-compressing-suture balancing element 590 to anterior portion 470.

Include a statement about removal of all sutures together after placement on the retina (all tied to the crossbar or equivalent). All the sutures can be tied to the bone, and at the end of the procedure the bone is freed and all the sutures are removed from the eye in one step.

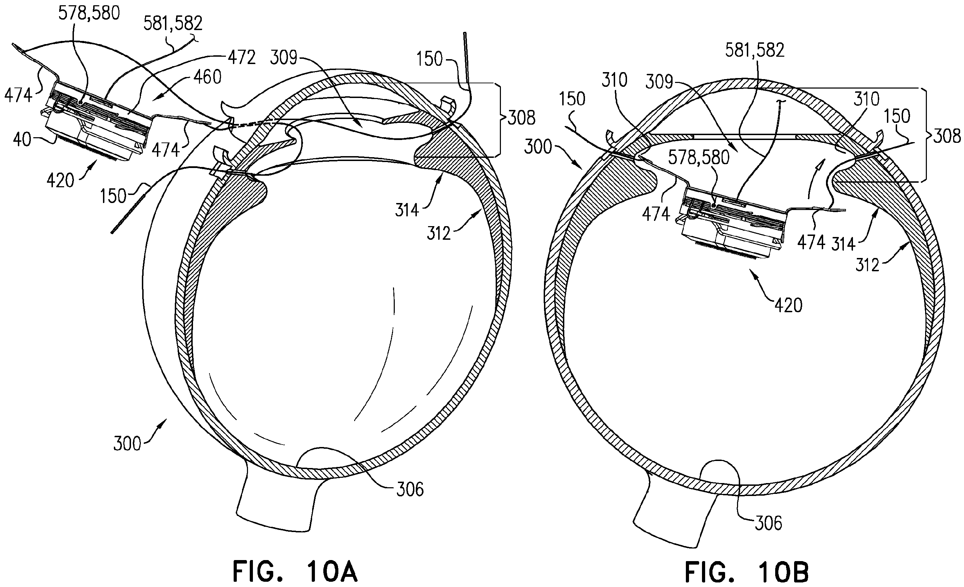

Figs. 10A-F are schematic illustrations of a surgical procedure for implantation of apparatus 420. Figs. 10A-F show a cross section of eye 300 and the deployment of apparatus 420 in eye 300 to yield the implantation and anchoring of retinal implant 40 on retina 306.

Reference is first made to Fig. 10A. As shown, apparatus 420 is inserted into eye 300 in a fully-compressed state of apparatus 420. During insertion of apparatus 420 into the eye, apparatus 420 is maintained in the fully-compressed state by first compressing element 578, e.g., first compressing suture 580, which secures anterior portion 470 with respect to posterior portion 490. In addition, as described hereinabove with reference to Figs. 9A-B, before insertion of apparatus 420 into the eye, second compressing element 581, e.g., second compressing suture 582, has been coupled to posterior portion 490, such as during manufacture of apparatus 420.

Apparatus 420 is typically inserted through a limbal incision in the eye, typically following removal of the native lens and vitreous body of the eye. For some applications, apparatus 420 is inserted through the limbal incision by grasping the apparatus by one of haptics 474 and pushing the apparatus through the limbal incision. Alternatively, an insertion tool is used to insert apparatus 420 through the limbal incision. For example, the insertion tool may comprise a cannula in which the apparatus is removably disposed. The cannula is inserted through the limbal incision and the apparatus is deployed from the cannula within the eye.

Reference is now made to Figs. 10B-C. Following insertion into the eye, apparatus 420 is anchored to anterior segment 308 of eye 300, typically within posterior chamber 309, typically against ciliary sulcus 310 of the eye. As mentioned above, for some applications, anchor 460 comprises two haptics 474, and each haptic 474 is placed against ciliary sulcus 310. For some applications, each haptic 474 is sutured to ciliary sulcus 310 by haptic-suture 150. Anchoring haptics 474 to ciliary sulcus 310 typically stabilizes apparatus 420 in the eye and allows subsequent full deployment of anchor 460 and stable implantation of retinal implant 40 on retina 306. Alternatively, even though anchor 460 is configured to anchor retinal implant 40 to the retina such that anterior portion 470 of anchor 460 is mounted against ciliary sulcus 310, apparatus 420 may instead, typically inadvertently, be anchored to the pars plana, depending, for example, on the skill of the surgeon.

Reference is now made to Fig. 10D, which is a schematic illustration of partial release of the compression of apparatus 420. Typically, subsequently to the anchoring of anterior portion 470 to ciliary sulcus 310 through haptics 474 (as shown in Fig. 10C), second compressing element 581, e.g., second compressing suture 582, is held generally taut by the surgeon, typically from outside the eye. (Second compressing suture 582 is shown in Figs. 10C-E passing through the limbal incision through which apparatus 420 was introduced at the step described hereinabove with reference to Fig. 10A.) While second compressing element 581 is held generally taut, a first portion of anchor 460 is released by separating anterior portion 470 of anchor 460 from posterior portion 490 of anchor 460. This separation is typically facilitated by removal of first compressing element 578, e.g., first compressing suture 580, e.g., by cutting first compressing suture 580 using cutting tool 190. Upon the cutting of first compressing element 578, e.g., first compressing suture 580, elastic element 482 is (a) partly released and (b) partly constrained by second compressing element 581, such that apparatus 420 remains in a partially-compressed state. As a result, a portion of elastic element 482 is extended longitudinally, posteriorly, into vitreous cavity 102 of eye 300. As shown in Fig. 10D, following the cutting of first compressing element 578, e.g., first compressing suture 580, an anterior portion of elastic element 482 is decompressed and a posterior portion of elastic element 482 remains compressed.

Typically, upon the above-described partial release of elastic element 482, a distance of anterior portion 470 of anchor 460 to posterior portion 490 of anchor 460, is 30 - 60%, e.g., 40%, of the distance of anterior portion 470 to posterior portion 490 of anchor 460 following deployment of apparatus 420 in the eye. In this partly-compressed state, a distance between anterior portion 470 and posterior portion 490 (a) is larger than a distance between anterior portion 470 and posterior portion 490 in the fully-compressed state of apparatus 420, and (b) is smaller than a distance between anterior portion 470 and posterior portion 490 in the expanded state, i.e., following deployment in the eye, of apparatus 420. Typically, in the partly-compressed state the distance of anterior portion 470 to posterior portion 490 is 8 - 15 mm.

Arrows 526 and 528 schematically indicate helical and posterior motion, respectively, of apparatus 420 following removal of first compressing element 578, e.g., first compressing suture 580, as apparatus 420 assumes the configuration shown in Fig. 10D. Alternatively, during the transition from the fully-compressed state to the partially-compressed state, apparatus 420 undergoes little or no helical motion, such as in configurations in which apparatus 420 comprises anchor 660, described hereinbelow with reference to Figs. 12A-B, instead of anchor 460.

As shown in Fig. 10D, subsequently to partial release of anchor 460 as shown in the transition from Fig. 10C to Fig. 10D, posterior portion 490 of anchor 460, e.g., posterior ring 492 and/or hooks or rings 588 of posterior portion 490, is grasped by grasping tool 196 in order to allow subsequent full release of the compression of apparatus 420 and lowering of retinal implant 40 on to retina 306 in a controlled manner.

Reference is now made to Fig. 10E, which is a schematic illustration of apparatus 420 following the full release of the compression of apparatus 420 and the deployment of apparatus 420 in eye 300. While second compressing element 581, e.g., second compressing suture 582, is still coupled to posterior portion 490 of apparatus 420, the tension in second compressing element 581 is gradually released and retinal implant 40 is lowered onto retina 306 by the surgeon, typically controlling the lowering using second compressing element 581 and/or grasping tool 196. Optionally, the surgeon may use grasping tool 196 to maneuver retinal implant 40 slightly sideways during lowering, such as in order to compensate for the slight tilt of the visual axis relative to the optical axis of the eye. Typically, retinal implant 40 is implanted in an epi-retinal position such that electrode array 120 contacts retina 306 and provides stimulation to retina 306. (For some applications, upon full deployment of apparatus 420, respective central axes of anterior ring 472 and posterior ring 492 are angled with respect to each other, e.g., by about 5 degrees, because the retinal implantation site on the macula is positioned on the visual axis which is tilted by about 5 degrees with respect to the optical axis of the eye.)

Reference is now made to Fig. 10F, which is a schematic illustration of apparatus 420 following (a) the full release of the compression of apparatus 420, (b) the removal of second compressing element 581, e.g., second compressing suture 582, and (c) insertion of IOL 100, in accordance with some applications of the present invention. For some applications, IOL 100 is inserted into the eye and positioned in anterior ring 472, following deployment of anchor 460 in the eye. For applications in which posterior connecting suture 586 is provided, such as described hereinabove with reference to Figs. 9A-B, posterior connecting suture 586 is removed upon or after removal of second compressing suture 582. For applications in which second-compressing-suture balancing element 590 is provided, such as described hereinabove with reference to Figs. 9A-B, second-compressing-suture balancing element 590 is removed, soon before or after removing second compressing suture 582.

As shown in Fig. 10F, when anchor 460 is fully deployed in eye 300, anterior portion 470 is anchored within posterior chamber 309, typically against ciliary sulcus 310, and elastic element 482 extends longitudinally from the anterior of eye 300 to the posterior of the eye, such that retinal implant 40, surrounded by posterior portion 490 of anchor 460, is placed on retina 306. For some applications, retinal implant 40 is placed on retina 306 such that electrodes 122 penetrate the retina. Alternatively, electrode array 120 comprises surface electrodes, which contact the retina but do not penetrate the retina. Posterior portion 490 of anchor 460 typically does not penetrate the retina.

In addition, when anchor 460 is deployed as shown in Fig. 10G, anchor 460 is in compression between the anterior and posterior of eye 300, and a distance between anterior ring 472 and posterior ring 492 (i.e., a length of elastic element 482), is shorter than the distance when apparatus 420 is unconstrained. Typically, when deployed in the eye, anchor 460 presses in a posterior direction against retinal implant 40 and in an anterior direction against ciliary sulcus 310, (e.g., via contact of haptics 474 with the ciliary sulcus).

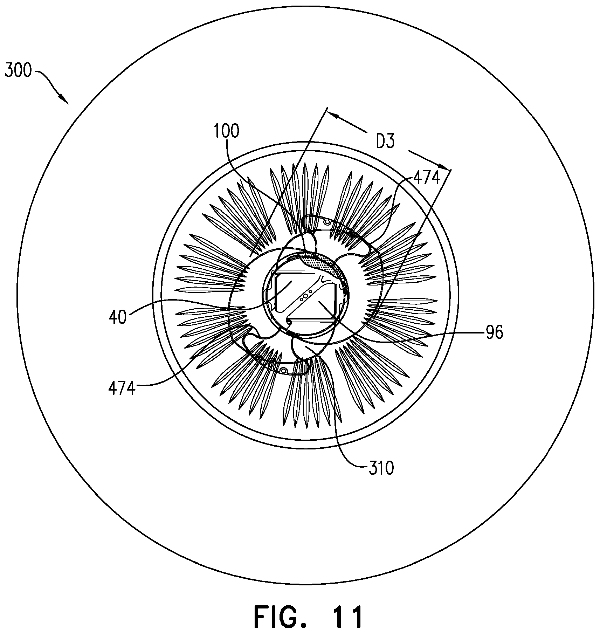

Reference is now made to Fig. 11, which is a schematic illustration of a front view of eye 300 having apparatus 420 fully deployed in the eye. As shown, anchor 460, when deployed in the eye, allows generally unobstructed passage of light into the eye and to retinal implant 40 (in particular, unobstructed passage of light to photosensors 96 of retinal implant 40).

Reference is now made to Figs. 12A-B, which are schematic illustrations of an anchor 660, in an unconstrained state, in accordance with some applications of the present invention. Other than as described below, anchor 660 is similar to anchor 460, described hereinabove with reference to Figs. 7A-9B, and may implement any features thereof, *mutatis mutandis;* apparatus 420 may comprise anchor 660 instead of anchor 460. Like reference numerals refer to like elements.

The features of anchor 660 may also be combined with the features of anchor 60, described hereinabove with reference to Figs. 1A-3, *mutatis mutandis* (including providing holes 84 in non-helical elastic element 682). Apparatus 20 may comprise an anchor with these combined features, instead of anchor 60, in which case apparatus 20 typically does not comprise rotational-force reduction element 68, described hereinabove with reference to Figs. 6A-B, because the rotational-force reduction element is unnecessary.

Anchor 660 is typically shaped to define (i) an anterior portion 670, (ii) a posterior portion 690 surrounding retinal implant 40 (not shown in Figs. 12A-B, but shown, for example, in Figs. 7A-B and 8), and (iii) a middle portion 680 extending longitudinally between anterior portion 670 and posterior portion 690. As shown in Figs. 12A-B, for some applications, anterior portion 670 comprises an anterior ring 672, posterior portion 690 comprises a posterior ring 692 surrounding retinal implant 40, and middle portion 680 comprises an elastic element 682 (e.g., a spring) extending longitudinally between anterior ring 672 and posterior ring 692. Elastic element 682 is non-helical.

Elastic element 682 is configured such that during the transition from the fully-compressed state to the partially-compressed state, such as described hereinabove with reference to Figs. 10C-D, posterior portion 690 (e.g., posterior ring 692) does not rotate or rotates only a small amount (e.g., less than 30 degrees) with respect to anterior portion 670 (e.g., anterior ring 672). Alternatively or additionally, elastic element 682 is configured such that during the transition from the fully-compressed state to the unconstrained, fully non-compressed state, shown in Figs. 12A-B, posterior portion 690 (e.g., posterior ring 692) does not rotate or rotates only a small amount (e.g., less than 30 degrees, typically less than 10 degrees) with respect to anterior portion 670 (e.g., anterior ring 672). Elastic element 682 typically has one or both of the above-mentioned rotational properties even if, during manufacture of anchor 660, during compression of anchor 660 from the unconstrained, fully non-compressed state to the fully-compressed state, posterior portion 690 (e.g., posterior ring 692) is not rotated with respect to anterior portion 670 (e.g., anterior ring 672).

For some applications, as shown Figs. 12A-B, elastic element 682 is shaped so as to have a zigzag shape. During manufacture of anchor 660, during compression of anchor 660 from the unconstrained, fully non-compressed state to the fully-compressed state, elastic element 682 folds like an accordion (rather than rotating). This accordion-folding may reduce the strength of elastic element 682, and, alternatively or additionally, may reduce the risk of the struts of elastic element 682 touching, snagging, and/or becoming entangled with one other during the transition to the non-compressed state in the eye.

Reference is still made to Figs. 12A-B. When expanded and unconstrained, elastic element 482 typically has an unconstrained length that is at least 20 mm and/or less than 40 mm, e.g., between 20 - 40 mm, e.g., between 18 - 35 mm. Typically, the length of elastic element 82 is also a distance D1 between anterior ring 472 and posterior ring 492 (e.g., between 13 and 23 mm). In this context, in the specification and in the claims, a "distance" refers to the distance from a first closest surface to a second closest surface. Elastic element 482 of anchor 460, described hereinabove with reference to Figs. 7A-9B, may also have these dimensions.

For some applications, techniques and apparatus described in the following patents and patent applications are combined with techniques and apparatus described herein: US Patent 8,150,526 to Gross et al.; US Patent 9,265,945 to Gross et al.; US Patent 8,718,784 to Gefen et al.; US Patent 8,428,740 to Gefen et al.; US Patent 8,442,641 to Gross et al.; US Patent 8,706,243 to Gefen et al.; US Patent 9,198,753 to Gefen et al.; US Patent 10,226,625 to Weinberger et al.; US Patent Application Publication 2018/0117329 to Degtiar et al.; and PCT Publication WO 2018/083699 to Degtiar et al. In case of conflict between the definitions used herein and those used in the patents and patent application publications listed above, the definitions used herein shall control.

## Claims

1. Apparatus (20, 420) comprising:
a retinal implant (40), configured for implantation on a retina (306) of a subject's eye (300) in an epi-retinal position; and
an anchor (60, 460, 660) configured to anchor the retinal implant (40) to the retina, **characterized in that**:
the anchor (60, 460, 660) is shaped to define (i) an anterior portion (70, 470, 670), (ii) a posterior portion (90, 490, 690) coupled to the retinal implant (40), and (iii) a middle portion (80, 480, 680) extending longitudinally between the anterior portion (70, 470, 670) and the posterior portion (90, 490, 690),
with the anchor (60, 460, 660) being configured to anchor the retinal implant (40) to the retina (306) such that when the retinal implant (40) is implanted on the retina (306), the anterior portion (70, 470, 670) of the anchor (60, 460, 660) is mounted against a ciliary sulcus (310) of the eye (300).

2. The apparatus (20, 420) according to claim 1, wherein (i) the anterior portion (70, 470, 670) of the anchor (60, 460, 660) comprises an anterior ring (72, 472, 672), (ii) the posterior portion (90, 490, 690) comprises a posterior ring (92, 492, 692) surrounding the retinal implant (40), and (iii) the middle portion (80, 480, 680) comprises an elastic element (82, 482, 682) extending longitudinally between the anterior ring (72, 472, 672) and the posterior ring (92, 492, 692).

3. The apparatus (20, 420) according to claim 2, wherein the elastic element (82, 482, 682) has an unconstrained length that is at least 13 mm.

4. The apparatus (20, 420) according to claim 3, wherein the unconstrained length of the elastic element (82, 482, 682) is at least 23 mm.

5. The apparatus (20, 420) according to claim 1,
wherein: (a) the apparatus (20, 420) is configured to be inserted into the eye (300) of the subject in a fully-compressed state of the apparatus (20, 420), and (b) the apparatus is configured to be in an expanded state of the apparatus (20, 420) when the posterior portion (90, 490, 690) is implanted on the retina (306) of the eye (300), and
wherein in the fully-compressed state of the apparatus (20, 420), the middle portion (80, 480, 680) is longitudinally compressed such that a distance between the anterior portion (70, 470, 670) and the posterior portion (90, 490, 690) is smaller than a distance between the anterior portion (70, 470, 670) and the posterior portion (90, 490, 690) in the expanded state.

6. The apparatus (20, 420) according to claim 5,
wherein the apparatus (20, 420) is further configured to assume a partly-compressed state following insertion into the eye (300) and prior to implantation on the retina (306), such that in the partly-compressed state a distance between the anterior portion (70, 470, 670) and the posterior portion (90, 490, 690) (a) is larger than a distance between the anterior portion (70, 470, 670) and the posterior portion (90, 490, 690) in the fully-compressed state and (b) is smaller than a distance between the anterior portion (70, 470, 670) and the posterior portion (90, 490, 690) in the expanded state, and
wherein the apparatus (20, 420) further comprises (i) a first compressing element (178, 578) configured to maintain compression of the apparatus (20, 420) by securing the anterior portion (70, 470, 670) with respect to the posterior portion (90, 490, 690) in the fully-compressed state of the apparatus (20, 420), and (b) a second compressing element (181, 581) coupled to the posterior portion (90, 490, 690) and configured to maintain compression of the apparatus (20, 420).

7. The apparatus (20, 420) according to claim 6,
wherein the middle portion (80, 480, 680) comprises an elastic element (82, 482, 682) extending longitudinally between the anterior portion (70, 470, 670) and the posterior portion (90, 490, 690), and
wherein the second compressing element (181, 581) is configured to maintain compression of the apparatus (20, 420) by securing a section of the elastic element (82, 482, 682) to the posterior portion (90, 490, 690) in the partly-compressed state of the apparatus (20, 420).

8. The apparatus (20, 420) according to claim 6, wherein the first (178, 578) and second (181, 581) compressing elements comprise first (180, 580) and second (182, 582) compressing sutures, respectively.

9. The apparatus (420) according to claim 8, further comprising a second-compressing-suture balancing element (590), which is removably coupled to the anterior portion (470), and which is shaped so as to define a central opening (592) through which the second compressing suture (582) passes.

10. The apparatus (20, 420) according to claim 6, wherein, in the expanded state the distance of the anterior portion (70, 470, 670) to the posterior portion (90, 490, 690) is 13 - 23 mm.

11. The apparatus (20, 420) according to any one of claims 1-10, further comprising an intraocular lens (IOL) (100) coupled to the anterior portion (70, 470, 670) of the anchor (60, 460, 660).

12. The apparatus (20, 420) according to any one of claims 1-10, further comprising at least one haptic (74, 474) coupled to the anterior portion (70, 470, 670) of the anchor (60, 460, 660) and configured to anchor the anterior portion (70, 470, 670) of the anchor (60, 460, 660) to the ciliary sulcus (310).

13. The apparatus (20, 420) according to claim 12, wherein the at least one haptic (74, 474) comprises a leading haptic and a trailing haptic.

14. The apparatus (20, 420) according to any one of claims 1-10, wherein the retinal implant (40) comprises an electrode array (120) comprising electrodes (122).

15. The apparatus (20, 420) according to claim 14, wherein the retinal implant (40) further comprises an array of photosensors (96); and driving circuitry (98), configured to receive signals from the photosensors (96) and in response thereto, to drive the electrodes (122) to apply currents to the retina (306).

## Patentansprüche

1. Vorrichtung (20, 420), umfassend:
ein Netzhautimplantat (40), das zur Implantation auf einer Netzhaut (306) eines Auges (300) eines Subjekts in einer epiretinalen Position konfiguriert ist; und
eine Verankerung (60, 460, 660), die dazu konfiguriert ist, das Netzhautimplantat (40) an der Netzhaut zu verankern, **dadurch gekennzeichnet, dass**:
die Verankerung (60, 460, 660) so geformt ist, dass sie (i) einen anterioren Abschnitt (70, 470, 670), (ii) einen posterioren Abschnitt (90, 490, 690), der an das Netzhautimplantat (40) gekoppelt ist, und (iii) einen mittleren Abschnitt (80, 480, 680), der sich in Längsrichtung zwischen dem anterioren Abschnitt (70, 470, 670) und dem posterioren Abschnitt (90, 490, 690) erstreckt, definiert,
wobei die Verankerung (60, 460, 660) dazu konfiguriert ist, das Netzhautimplantat (40) an der Netzhaut (306) zu verankern, sodass, wenn das Netzhautimplantat (40) auf der Netzhaut (306) implantiert ist, der anteriore Abschnitt (70, 470, 670) der Verankerung (60, 460, 660) gegen einen Sulcus ciliaris (310) des Auges (300) montiert ist.

2. Vorrichtung (20, 420) nach Anspruch 1, wobei (i) der anteriore Abschnitt (70, 470, 670) der Verankerung (60, 460, 660) einen anterioren Ring (72, 472, 672) umfasst, (ii) der posteriore Abschnitt (90, 490, 690) einen posterioren Ring (92, 492, 692) umfasst, der das Netzhautimplantat (40) umgibt, und (iii) der mittlere Abschnitt (80, 480, 680) ein elastisches Element (82, 482, 682) umfasst, das sich in Längsrichtung zwischen dem anterioren Ring (72, 472, 672) und dem posterioren Ring (92, 492, 692) erstreckt.

3. Vorrichtung (20, 420) nach Anspruch 2, wobei das elastische Element (82, 482, 682) eine freie Länge aufweist, die mindestens 13 mm beträgt.

4. Vorrichtung (20, 420) nach Anspruch 3, wobei die freie Länge des elastischen Elements (82, 482, 682) mindestens 23 mm beträgt.

5. Vorrichtung (20, 420) nach Anspruch 1,
wobei: (a) die Vorrichtung (20, 420) dazu konfiguriert ist, in einem vollständig komprimierten Zustand der Vorrichtung (20, 420) in das Auge (300) des Subjekts eingeführt zu werden, und (b) die Vorrichtung dazu konfiguriert ist, sich in einem expandierten Zustand der Vorrichtung (20, 420) zu befinden, wenn der posteriore Abschnitt (90, 490, 690) auf der Netzhaut (306) des Auges (300) implantiert wird, und
wobei im vollständig komprimierten Zustand der Vorrichtung (20, 420) der mittlere Abschnitt (80, 480, 680) in Längsrichtung komprimiert ist, sodass ein Abstand zwischen dem anterioren Abschnitt (70, 470, 670) und dem posterioren Abschnitt (90, 490, 690) kleiner ist als ein Abstand zwischen dem anterioren Abschnitt (70, 470, 670) und dem posterioren Abschnitt (90, 490, 690) im expandierten Zustand.

6. Vorrichtung (20, 420) nach Anspruch 5,
wobei die Vorrichtung (20, 420) ferner dazu konfiguriert ist, nach der Einführung in das Auge (300) und vor der Implantation auf der Netzhaut (306) einen teilweise komprimierten Zustand anzunehmen, sodass im teilweise komprimierten Zustand ein Abstand zwischen dem anterioren Abschnitt (70, 470, 670) und dem posterioren Abschnitt (90, 490, 690) (a) größer ist als ein Abstand zwischen dem anterioren Abschnitt (70, 470, 670) und dem posterioren Abschnitt (90, 490, 690) im vollständig komprimierten Zustand und (b) kleiner ist als ein Abstand zwischen dem anterioren Abschnitt (70, 470, 670) und dem posterioren Abschnitt (90, 490, 690) im expandierten Zustand, und
wobei die Vorrichtung (20, 420) ferner (i) ein erstes Kompressionselement (178, 578), das dazu konfiguriert ist, die Kompression der Vorrichtung (20, 420) aufrechtzuerhalten, indem es den anterioren Abschnitt (70, 470, 670) in Bezug auf den posterioren Abschnitt (90, 490, 690) im vollständig komprimierten Zustand der Vorrichtung (20, 420) sichert, und (b) ein zweites Kompressionselement (181, 581), das an den posterioren Abschnitt (90, 490, 690) gekoppelt ist und dazu konfiguriert ist, die Kompression der Vorrichtung (20, 420) aufrechtzuerhalten, umfasst.

7. Vorrichtung (20, 420) nach Anspruch 6,
wobei der mittlere Abschnitt (80, 480, 680) ein elastisches Element (82, 482, 682) umfasst, das sich in Längsrichtung zwischen dem anterioren Abschnitt (70, 470, 670) und dem posterioren Abschnitt (90, 490, 690) erstreckt, und
wobei das zweite Kompressionselement (181, 581) dazu konfiguriert ist, die Kompression der Vorrichtung (20, 420) aufrechtzuerhalten, indem es einen Bereich des elastischen Elements (82, 482, 682) im teilweise komprimierten Zustand der Vorrichtung (20, 420) an dem posterioren Abschnitt (90, 490, 690) sichert.

8. Vorrichtung (20, 420) nach Anspruch 6, wobei das erste (178, 578) und zweite (181, 581) Kompressionselement eine erste (180, 580) bzw. zweite (182, 582) Kompressionsnaht umfassen.

9. Vorrichtung (420) nach Anspruch 8, ferner umfassend ein Ausgleichselement (590) der zweiten Kompressionsnaht, das entfernbar an den anterioren Abschnitt (470) gekoppelt ist und das so geformt ist, dass es eine zentrale Öffnung (592) definiert, durch die die zweite Kompressionsnaht (582) verläuft.

10. Vorrichtung (20, 420) nach Anspruch 6, wobei im expandierten Zustand der Abstand des anterioren Abschnitts (70, 470, 670) zum posterioren Abschnitt (90, 490, 690) 13-23 mm beträgt.

11. Vorrichtung (20, 420) nach einem der Ansprüche 1-10, ferner umfassend eine Intraokularlinse (IOL) (100), die an den anterioren Abschnitt (70, 470, 670) der Verankerung (60, 460, 660) gekoppelt ist.

12. Vorrichtung (20, 420) nach einem der Ansprüche 1-10, ferner umfassend mindestens eine Haptik (74, 474), die an den anterioren Abschnitt (70, 470, 670) der Verankerung (60, 460, 660) gekoppelt ist und dazu konfiguriert ist, den anterioren Abschnitt (70, 470, 670) der Verankerung (60, 460, 660) an dem Sulcus ciliaris (310) zu verankern.

13. Vorrichtung (20, 420) nach Anspruch 12, wobei die mindestens eine Haptik (74, 474) eine vordere Haptik und eine hintere Haptik umfasst.

14. Vorrichtung (20, 420) nach einem der Ansprüche 1-10, wobei das Netzhautimplantat (40) eine Elektrodenanordnung (120) umfasst, die Elektroden (122) umfasst.

15. Vorrichtung (20, 420) nach Anspruch 14, wobei das Netzhautimplantat (40) ferner eine Anordnung von Fotosensoren (96); und eine Ansteuerschaltung (98) umfasst, die dazu konfiguriert ist, Signale von den Fotosensoren (96) zu empfangen und als Reaktion darauf die Elektroden (122) anzusteuern, um Ströme an die Netzhaut (306) anzulegen.

## Revendications

1. Appareil (20, 420) comprenant :
un implant rétinien (40), conçu pour une implantation sur la rétine (306) de l'œil d'un sujet (300) dans une position épi-rétinienne ; et
un dispositif d'ancrage (60, 460, 660) conçu pour ancrer l'implant rétinien (40) à la rétine, **caractérisé en ce que** :
le dispositif d'ancrage (60, 460, 660) est profilé pour définir (i) une partie antérieure (70, 470, 670), (ii) une partie postérieure (90, 490, 690) couplée à l'implant rétinien (40), et (iii) une partie médiane (80, 480, 680) s'étendant longitudinalement entre la partie antérieure (70, 470, 670) et la partie postérieure (90, 490, 690),
le dispositif d'ancrage (60, 460, 660) étant conçu pour ancrer l'implant rétinien (40) à la rétine (306) de sorte que lorsque l'implant rétinien (40) est implanté sur la rétine (306), la partie antérieure (70, 470, 670) du dispositif d'ancrage (60, 460, 660) est montée contre un sillon ciliaire (310) de l'œil (300).

2. Appareil (20, 420) selon la revendication 1, (i) ladite partie antérieure (70, 470, 670) du dispositif d'ancrage (60, 460, 660) comprenant un anneau antérieur (72, 472, 672), (ii) ladite partie postérieure (90, 490, 690) comprenant un anneau postérieur (92, 492, 692) qui entoure l'implant rétinien (40), et (iii) la partie médiane (80, 480, 680) comprenant un élément élastique (82 , 482, 682) qui s'étend longitudinalement entre l'anneau antérieur (72, 472, 672) et l'anneau postérieur (92, 492, 692).

3. Appareil (20, 420) selon la revendication 2, ledit élément élastique (82, 482, 682) présentant une longueur libre de contrainte qui est d'au moins 13 mm.

4. Appareil (20, 420) selon la revendication 3, ladite longueur libre de contrainte de l'élément élastique (82, 482, 682) étant d'au moins 23 mm.

5. Appareil (20, 420) selon la revendication 1 :
(a) ledit appareil (20, 420) étant conçu pour être inséré dans l'œil (300) du sujet dans un état complètement comprimé de l'appareil (20, 420), et (b) l'appareil étant conçu pour se trouver dans un état déployé de l'appareil (20, 420) lorsque la partie postérieure (90, 490, 690) est implantée sur la rétine (306) de l'œil (300), et
dans l'état complètement comprimé de l'appareil (20, 420), la partie médiane (80, 480, 680) étant comprimée longitudinalement de sorte que la distance entre la partie antérieure (70, 470, 670) et la partie postérieure (90, 490, 690) soit plus petite que la distance entre la partie antérieure (70, 470, 670) et la partie postérieure (90, 490, 690) dans l'état déployé.

6. Appareil (20, 420) selon la revendication 5,
ledit appareil (20, 420) étant en outre conçu pour adopter un état partiellement comprimé après insertion dans l'œil (300) et avant implantation sur la rétine (306), de sorte que dans l'état partiellement comprimé la distance entre la partie antérieure (70, 470, 670) et la partie postérieure (90, 490, 690) (a) soit plus grande que la distance entre la partie antérieure (70, 470, 670) et la partie postérieure (90, 490, 690) dans l'état complètement comprimé et (b) soit plus petite que la distance entre la partie antérieure (70, 470, 670) et la partie postérieure (90, 490, 690) dans l'état déployé, et
ledit appareil (20, 420) comprenant en outre (i) un premier élément de compression (178, 578) conçu pour maintenir la compression de l'appareil (20, 420) en fixant la partie antérieure (70, 470, 670) par rapport à la partie postérieure (90, 490, 690) dans l'état complètement comprimé de l'appareil (20, 420), et (b) un second élément de compression (181, 581) couplé à la partie postérieure (90, 490, 690) et conçu pour maintenir la compression de l'appareil (20, 420).

7. Appareil (20, 420) selon la revendication 6,
ladite partie médiane (80, 480, 680) comprenant un élément élastique (82, 482, 682) qui s'étend longitudinalement entre la partie antérieure (70, 470, 670) et la partie postérieure (90, 490, 690), et
ledit second élément de compression (181, 581) étant conçu pour maintenir la compression de l'appareil (20, 420) en fixant une section de l'élément élastique (82, 482, 682) à la partie postérieure (90, 490, 690) dans l'état partiellement comprimé de l'appareil (20, 420).

8. Appareil (20, 420) selon la revendication 6, lesdits premier (178, 578) et second (181, 581) éléments de compression comprenant des première (180, 580) et seconde (182, 582) sutures de compression, respectivement.

9. Appareil (420) selon la revendication 8, comprenant en outre un second élément d'équilibrage de suture de compression (590), qui est couplé de manière amovible à la partie antérieure (470), et qui est formé de manière à définir une ouverture centrale (592) à travers laquelle passe la seconde suture de compression (582).

10. Appareil (20, 420) selon la revendication 6, dans l'état déployé, la distance de la partie antérieure (70, 470, 670) à la partie postérieure (90, 490, 690) étant de 13 à 23 mm.

11. Appareil (20, 420) selon l'une quelconque des revendications 1 à 10, comprenant en outre une lentille intraoculaire (IOL) (100) couplée à la partie antérieure (70, 470, 670) du dispositif d'ancrage (60, 460, 660).

12. Appareil (20, 420) selon l'une quelconque des revendications 1 à 10, comprenant en outre au moins une haptique (74, 474) couplée à la partie antérieure (70, 470, 670) du dispositif d'ancrage (60, 460, 660) et conçue pour ancrer la partie antérieure (70, 470, 670) du dispositif d'ancrage (60, 460, 660) au sillon ciliaire (310).

13. Appareil (20, 420) selon la revendication 12, ladite au moins une haptique (74, 474) comprenant une haptique avant et une haptique arrière.

14. Appareil (20, 420) selon l'une quelconque des revendications 1 à 10, ledit implant rétinien (40) comprenant un réseau d'électrodes (120) qui comprend des électrodes (122).

15. Appareil (20, 420) selon la revendication 14, ledit implant rétinien (40) comprenant en outre un réseau de photocapteurs (96) ; et un circuit de commande (98), configuré pour recevoir les signaux provenant des photocapteurs (96) et, en réponse à ceux-ci, pour commander aux électrodes (122) d'appliquer des courants sur la rétine (306).
